# EUROPÄISCHE PATENTANMELDUNG

(11) **EP 1 212 977 A1**
(43) Veröffentlichungstag der Anmeldung: **12.06.2002**
(21) Anmeldenummer: 00127098.2
(22) Anmeldetag: 11.12.2000
(51) Int. Cl.: A61B 5/00

(54) **Verfahren und Vorrichtung zur Bestimmung der Temperaturverteilung an der Körperoberfläche einer Person**

(71) Anmelder: Berz, Reinhold, 88430 Rot a. d. Rot (DE)
(72) Erfinder: Berz, Reinhold, 88430 Rot a. d. Rot (DE)
(74) Vertreter: Lucht, Silvia, Dipl.-Phys.

(57) **Zusammenfassung**

Es werden ein Verfahren und eine Vorrichtung zur wierderholten Bestimmung der Temperaturverteilung an der Körperoberfläche einer Person unter reproduzierbaren Bedingungen im Rahmen der Regulationsthermographie vorgeschlagen, wobei mit Hilfe einer Infrarot-Kamera eine erste Temperaturverteilung des Körpers oder von Teilen des Körpers der Person aufgenommen wird. Nachdem die Person einem Belastungsreiz ausgesetzt wurde, wird eine zweite Aufnahme der Temperaturverteilung des Körpers der Person mit Hilfe der Infrarot-Kamera durchgefürt.

## Beschreibung

### Stand der Technik

Die Erfindung geht aus von einem Verfahren und einer Vorrichtung zur Bestimmung der Temperaturverteilung an der Körperoberfläche einer Person im Rahmen der Regulationsthermographie nach dem Oberbegriff des Anspruchs 1 und des Anspruchs 13.

Bei der Regulationsthermographie wird die Temperatur an vielen einzelnen Meßpunkten des Körpers einer Person nach einem vorgegebenen Schema bestimmt. Dabei werden die Temperaturen einmal vor und einmal nach einem Belastungsreiz der üblicherweise in einer sanften Abkühlung des Patienten besteht, gemessen. Hierzu muß sich die Person ca. 10 min mit entblößtem Oberkörper bei einer Raumtemperatur von ca. 20°C abkühlen. Durch diesen großflächigen Abkühlungsreiz wird der Körper zu einer Wärmeregulation veranlaßt, deren Ergebnis die zweite Temperaturmessung erfaßt. Aus den Differenzen zwischen den ersten und zweiten Temperaturmeßwerten können diagnostische Hinweise auf die allgemeine Reaktionsfähigkeit und auf einzelne lokale Störungen gewonnen werden. Die Temperaturmessungen werden entweder als Kontaktmessung durch Aufsetzen eines Meßfühlers auf die Körperoberfläche der Person oder als Strahlungsmessung mit Hilfe von Infrarotfühlern durchgeführt. Aus den zahlreichen über den gesamten Körper verteilten Meßpunkten ergibt sich ein Temperaturmuster der Körperoberfläche. Aus diesem Temperaturmuster werden Rückschlüsse auch auf die zwischen den Meßpunkten herrschende Temperatur gezogen.

Als nachteilig erweist sich bei diesem bekannten Verfahren, daß die Meßpunkte der ersten und zweiten Temperaturmessung nicht eindeutig übereinstimmen, da die Meßpunkte von der die Messung durchführenden Person innerhalb der vorgegebenen Meßareale frei gewählt werden. Darüber hinaus werden nicht der gesamte Körper, sondern lediglich einzelne Meßpunkte auf der Körperoberfläche erfaßt. Temperaturabweichungen die zwischen den Meßpunkten liegen, können somit bei der Messung übersehen werden.

### Die Erfindung und ihre Vorteile

Demgegenüber haben das erfindungsgemäße Verfahren mit den kennzeichnenden Merkmalen des Anspruchs 1 und die erfindungsgemäße Vorrichtung mit den kennzeichnenden Merkmalen des Anspruchs 13 den Vorteil, daß mittels einer Infrarot-Kamera die gesamte Körperoberfläche oder große Teile der Körperoberfläche erfaßt werden. Es ergibt sich damit eine Temperaturverteilung der gesamten aufgenommenen Körperoberfläche und nicht nur einzelner Meßpunkte wie beim Stand der Technik. Ein Vergleich der aufgenommenen Temperaturverteilung wird erleichtert, da besonders charakteristische Merkmale in den aufgenommenen Temperaturverteilungen zur Auswertung herangezogen werden können. Die die Regulationsthermographie durchführende Person muß nicht die vorgeschriebenen Meßpunkte kennen. Mit der Infrarot-Kamera werden lediglich von einem Teil des Körpers Aufnahmen gemacht.

Zwischen der ersten und zweiten Temperaturaufnahme wird die Person einem Belastungsreiz ausgesetzt. Dieser kann beispielsweise in einer großflächigen oder punktuellen Temperaturreizung, dem Verabreichen eines Medikaments oder einer sportlichen Betätigung bestehen. Hierzu kann die Person nach Durchführen der ersten Temperaturaufnahme die zur Messung eingenommene Position verlassen, ohne daß dabei eine Verfälschung oder eine Ungenauigkeit der Ergebnisse befürchtet werden muß.

Nach einer vorteilhaften Ausgestaltung der Erfindung werden zum einen der Abstand zwischen Infrarot-Kamera und der Person und zum anderen der Winkel der Person bezüglich der Verbindung zur Infrarot-Kamera eingestellt. Die Aufnahmen der Temperaturverteilung erfolgen damit unter reproduzierbaren Bedingungen. Hierzu wird der Abstand zwischen der Infrarot-Kamera und der sich der Messung unterziehenden Person bestimmt und fest vorgegeben. Die Kamera wird ortsfest in einer ebenfalls vorgegebenen Höhe angeordnet. Darüber hinaus wird der Winkel zwischen Kamera und Person eingestellt. Damit ist der Winkel zwischen der Verbindungslinie Kamera und Person einerseits und der Blickrichtung der Person andererseits gemeint. Zur Durchführung der Messung kann die Person entweder stehen oder sitzen. Da die Ortsparameter von Kamera und Person vorgegeben und reproduzierbar wieder einstellbar sind, kann die Person nach Durchführen der ersten Temperaturaufnahme die zur Messung eingenommene Position verlassen, ohne daß dabei eine Verfälschung oder eine Ungenauigkeit der Ergebnisse befürchtet werden muß. Zur Durchführung der zweiten Temperaturaufnahme werden die Höhe der Kamera, der Abstand zwischen Kamera und Person und der Winkel der Person wieder so eingestellt wie bei der ersten Temperaturmessung. Die Positionen der Meßpunkte in der ersten und der zweiten Temperaturaufnahme stimmen somit überein. Ein exakter Vergleich der Temperaturen identischer Meßpunkte ist somit möglich. Die aufgenommenen Temperaturverteilungen können in einen Computer eingegeben werden, um die Auswertung oder den Vergleich zu erleichtern.

Nach einer weiteren vorteilhaften Ausgestaltung der Erfindung werden die aufgenommenen Temperaturverteilungen in einen Computer eingegeben. Mithilfe des Computers wird die zweite Temperaturverteilung anhand charakteristischer Merkmale der ersten Temperaturverteilung ausgerichtet. Bei den charakteristischen Merkmalen kann er sich beispielsweise um die Kontur oder die Sihouette des Körpers oder Körperteils oder um besonders kalte oder warme Bereiche in der Aufnahme der ersten Temperaturverteilung handeln. Anhand dieser Merkmale kann die zweite Temperaturverteilung so ausgerichtet werden, daß übereinstimmende Bereiche oder Punkte des Körpers der Person hinsichtlich ihrer Temperatur in der ersten und der zweiten Temperaturverteilung verglichen werden können. Hierzu ist der Computer mit einer geeigneten Software ausgestattet. Die Ausrichtung kann entweder automatisch durch den Computer erfolgen oder durch den Bearbeiter, der die beiden auf dem Bildschirm des Computers angezeigten Temperaturverteilungen solange verschiebt und anpaßt, bis er sie übereinanderlegen kann. Erfolgt die Anpassung der Aufnahmen mit Hilfe des Computers, so sind keine Vorrichtungen zur Bestimmung der räumlichen Parameter der Infrarot-Kamera und der Person notwendig. Die Person kann sich zwischen den beiden Aufnahmen bewegen und die Einstellungen der ersten Aufnahmen verändern.

Nach einer vorteilhaften Ausgestaltung des erfindungsgemäßen Verfahrens können nicht nur zwei sondern auch drei oder mehr Temperaturaufnahmen durchgeführt werden. Zwischen je zwei Aufnahmen kann die Person einem erneuten Belastungsreiz ausgesetzt werden.

Nach einer weiteren vorteilhaften Ausgestaltung der Erfindung werden in den Aufnahmen einzelne Bereiche markiert und miteinander verglichen. Zur Durchführung des Vergleichs können die Aufnahmen übereinandergelegt oder übereinander projiziert werden. Dies ist insbesondere bei den in den Computer eingelesenen Aufnahmen von Vorteil. Auf diese Weise können Temperaturunterschiede zwischen zwei Aufnahmen am Bildschirm des Computers optisch erfaßt werden. Darüber hinaus können die Temperaturunterschiede identischer Meßpunkte zweier Temperaturaufnahmen auch rechnerisch bestimmt werden. Hierzu werden die Temperaturwerte zweier Aufnahmen mit Hilfe des Computers bestimmt und voneinander abgezogen. Hierzu können auch Durchschnittswerte der markierten Bereiche gebildet werden. Besonders einfach ist der Vergleich der Durchschnittswerte der markierten Bereiche verschiedener Aufnahmen. Die Durchschnittswerte können auch graphisch beispielsweise durch ein Diagramm dargestellt werden.

Die erfindungsgemäße Vorrichtung sieht eine Infrarot-Kamera mit Stativ zum ortsfesten Anordnen vor. Zur Abstandsmessung können beispielsweise Maßbänder oder Laserentfernungsmeßgeräte verwendet werden. Zur Einstellung des Winkels wird die Person auf einem Drehteller oder Drehschemel mit Gradeinteilung angeordnet. Um die Position der Person relativ zum Drehtisch oder Drehschemel fest vorzugeben, kann eine Vorrichtung zur Arretierung des Beckens der Person vorgesehen sein. Die Auswertung der Meßergebnisse wird durch einen Computer erleichtert. Hierzu ist auch eine spezielle Software vogesehen. Die Meßergebnisse können entweder in geeigneter Weise auf dem Bildschirm des Computers dargestellt oder mit Hilfe eines Druckers ausgedruckt werden. Zur Archivierung der Meßergebnisse eignen sich Datenträger.

Weitere Vorteile und vorteilhafte Ausgestaltungen der Erfindung sind den Ansprüchen entnehmbar.

## Patentansprüche

1. Verfahren zur wiederholten Bestimmung der Temperaturverteilung an der Körperoberfläche einer Person unter reproduzierbaren Bedingungen im Rahmen der Regulationsthermographie
**dadurch gekennzeichnet,**
**daß** mithilfe einer Infrarot-Kamera eine erste Temperaturverteilung des Körpers oder Teile des Körpers der Person aufgenommen wird,
**daß** die Person einem Belastungsreiz ausgesetzt wird,
**daß** mithilfe der Infrarot-Kamera eine zweite Temperaturverteilung des Körpers oder Teile des Körpers aufgenommen wird.

2. Verfahren nach Anspruch 1, **dadurch gekennzeichnet,**
**daß** die Person in einem vorgegebenen Abstand und unter einem vorgegebenen Winkel bezüglich einer ortsfesten Infrarot-Kamera angeordnet wird,
**daß** mithilfe der Infrarot-Kamera eine erste Temperaturverteilung des Körpers oder Teile des Körpers der Person aufgenommen wird,
**daß** die Person einem Belastungsreiz ausgesetzt wird,
**daß** die Person erneut in dem vorgegebenen Abstand und unter dem vorgegebenen Winkel bezüglich der ortsfesten Kamera angeordnet wird, und
**daß** mithilfe der Infrarot-Kamera eine zweite Temperaturverteilung des Körpers oder Teile des Körpers aufgenommen wird.

3. Verfahren nach Anspruch 2, **dadurch gekennzeichnet, daß** die Aufnahmen der Infrarot-Kamera in einen Computer eingegeben werden.

4. Verfahren nach Anspruch 1, **dadurch gekennzeichnet, daß** die aufgenommenen Temperaturverteilungen in einen Computer eingegeben werden, daß mithilfe des Computers die zweite Temperaturverteilung anhand charakteristischer Merkmale der ersten Temperaturverteilung ausgerichtet wird.

5. Verfahren nach Anspruch 4, **dadurch gekennzeichnet, daß** die Kontur der Aufnahme der ersten Temperaturverteilung in die Aufnahme der zweiten Temperaturverteilung eingeblendet wird.

6. Verfahren nach einem der vorhergehenden Ansprüche, **dadurch gekennzeichnet, daß** die Person mindestens einem weiteren Belastungsreiz ausgesetzt wird, und daß mithilfe der Infrarot-Kamera eine weitere Temperaturverteilung des Körpers oder Teile des Körpers aufgenommen wird.

7. Verfahren nach einem der vorhergehenden Ansprüche, **dadurch gekennzeichnet, daß** in den Aufnahmen Bereiche markiert und verglichen werden.

8. Verfahren nach Anspruch 7, **dadurch gekennzeichnet, daß** die markierten Bereiche verschiedener Aufnahmen zum Vergleich übereinanprojiziert werden.

9. Verfahren nach Anspruch 7 und 8, **dadurch gekennzeichnet, daß** die den markierten Bereichen zweier Aufnahmen zugeordneten Temperaturwerte mithilfe des Computers ermittelt werden, und
daß zum Vergleich die denselben Positionen zugeordneten Temperaturwerte zweier Aufnahmen vorneinander abgezogen werden.

10. Verfahren nach Anspruch 9, **dadurch gekennzeichnet, daß** aus den Temperaturwerten der Bereiche Durchschnittswerte gebildet werden.

11. Verfahren nach Anspruch 10, **dadurch gekennzeichnet, daß** die Durchschnittswerte der markierten Bereiche verschiedener Aufnahmen graphisch dargestellt werden.

12. Verfahren nach Anspruch 11, **dadurch gekennzeichnet, daß** die Durchschnittswerte mehrerer markierter Bereiche nebeneinander graphisch dargestellt werden.

13. Vorrichtung zur wiederholten Bestimmung der Temperaturverteilung an der Körperoberfläche einer Person unter reproduzierbaren Bedingungen im Rahmen der Regulationsthermographie, insbesondere zur Durchführung des Verfahrens nach einem der Ansprüche 1 oder 2,
**dadurch gekennzeichnet,**
**daß** eine Infrarot-Kamera vorgesehen ist,
**daß** ein Stativ zum ortsfesten Anordnen der Infrarot-Kamera vorgesehen ist,
**daß** eine Vorrichtung zur Abstandsmessung vorgesehen ist,
**daß** eine Vorrichtung zum Anordnen einer Person unter einem vorgegebenen Winkel zur Infrarot-Kamera vorgesehen ist.

14. Vorrichtung nach Anspruch 13, **dadurch gekennzeichnet, daß** ein Computer vorgesehen ist, in welchen die Aufnahmen der Infrarot-Kamera eingegeben werden können.

15. Vorrichtung nach Anspruch 13 oder 14, **dadurch gekennzeichnet, daß** eine Vorrichtung zur Arretierung des Beckens der Person vorgesehen ist.
